# EUROPÄISCHE PATENTSCHRIFT

(11) **EP 0 484 458 B1**
(45) Veröffentlichungstag und Bekanntmachung des Hinweises auf die Patenterteilung: **30.03.1994**
(21) Anmeldenummer: 90914162.4
(22) Anmeldetag: 01.08.1990
(51) Int. Cl.: A61B 1/12, A61B 1/30

(54) **ENDOSKOPVORRICHTUNG**
ENDOSCOPE DEVICE
ENDOSCOPE

(30) Priorität: 01.08.1989 DE 3925484
(43) Veröffentlichungstag der Anmeldung: 13.05.1992
(73) Patentinhaber: STM Medizintechnik Starnberg GmbH, 82319 Starnberg (DE)
(72) Erfinder: BOB, Konstantin, D-69469 Weinheim (DE); BOB, Alexander, D-69469 Weinheim (DE); GRUENDL, Andreas, D-81377 München (DE)
(74) Vertreter: Klunker . Schmitt-Nilson . Hirsch
(86) Internationale Anmeldenummer: EP9001264
(87) Internationale Veröffentlichungsnummer: WO9101677

(56) Entgegenhaltungen:
- DE-A- 3 630 660
- DE-C- 2 823 025
- US-A- 3 757 788
- US-A- 4 207 872

## Beschreibung

Die Erfindung bezieht sich auf eine Endoskopvorrichtung, die ein Endoskop und Mittel zum Hineinbewegen des Endoskops in einen kanalartigen Hohlraum mittels Fluiddrucks aufweist. Die Erfindung bezieht sich ferner auf eine Vorrichtung zum Einführen eines Endoskops in einen kanalartigen Hohlraum mittels Fluiddrucks, also die Mittel zum Hineinbewegen des Endoskops in den kanalartigen Hohlraum, ohne daß das eigentliche Endoskop Bestandteil der Gesamtvorrichtung ist.

Die Erfindung ist zur Anwendung auf medizinischem Gebiet, insbesondere zur Exploration von Hohlräumen oder röhrenartigen Kanälen des Körpers, sowie auf anderen Gebieten, insbesondere zur Exploration von Kohlräumen oder röhrenartigen Kanälen technischer Einrichtungen, bestimmt. Die nachfolgende Beschreibung geht primär auf das Gebiet der Medizin ein.

Auf medizinischem Gebiet haben sich Endoskope besonders eingeführt zur Exploration der Speiseröhre, des Magens, des Zwölffingerdarms vom Magen aus, des Darms vom Anus aus, der Harnröhre, der Blase und der Harnleiter. Endoskope sind an ihrem Vorderende mit einer Beleuchtungseinrichtung und mit einer Optik zur visuellen Erfassung des davorliegenden Bereichs des Körperhohlraums oder Körperkanals ausgerüstet. Während bis vor kurzem die vor dem Vorderende des Endoskops erfaßte, optische Information normalerweise mittels einer Faseroptik durch das Endoskop nach hinten zu seinem Bedienungsende übertragen worden ist, stellt jetzt der Einbau eines Kamerachips am vorderen Endoskopende und die elektrische Bildübertragung und die Darstellung der gewonnenen, optischen Information auf einem Bildschirmmonitor den neuesten Stand der Technik dar. Endoskope weisen ferner in der Regel einen sogenannten Arbeitskanal auf, durch den diverse Arbeitselemente eingeführt und bedient werden können, z.B. kleine Zangen zur Entnahme von Gewebeproben, Biopsienadeln, beheizbare Schneiddrähte, kleine Scheren, Koagulationselektroden oder dergleichen.

Schließlich sind in der Regel ein Fluidkanal für Spülflüssigkeit und Bedienungsdrähte zum Abwinkeln des Endoskopvorderendes in mehrere Richtungen vorhanden. Insgesamt hat das Endoskop, abgesehen von seinem hinteren Bedienungsende und einem Anschlußstrang, eine langgestreckte, biegsam-stabförmige Gestalt. Übliche Außendurchmesser liegen etwa im Bereich von 9 bis 15 mm, am vorderen Kopf etwas größer.

Bisher werden Endoskope dadurch in den Körper eingeführt, daß der Arzt von dem aus dem Körper herausragenden Teil des Endoskops her das drucksteife Endoskop in den Körper hineinschiebt. Diese Art des Einführens des Endoskops ist besonders mühsam, schwierig und zeitraubend beim Koloskop, insbesondere weil der Darm Abbiegungen und häufig Engstellen aufweist. Demzufolge gehören koloskopische Untersuchungen bisher zu den aufwendigen und für den Patienten unangenehmen Untersuchungen und kommen deshalb für eine breite Anwendung kaum in Betracht. Das Umgehen mit einem Koloskop erfordert einen hierin erfahrenen Arzt.

Diese Situation ist besonders nachteilig, weil Anomalien der Darmwand, beispielsweise Polypen, Adenome und Karzinome, in vielen Gegenden der Welt immer zahlreicher werden und weil eine möglichst frühzeitige Erkennung die Heilungschancen für den betreffenden Patienten ganz erheblich erhöht bzw. zu einer erheblichen Lebensverlängerung führt. Insofern ist es äußerst wünschenswert, ein Endoskop zur Verfügung zu haben, das unkomplizierter und schneller einführbar ist, auch von einschlägig nicht so erfahrenen Ärzten oder Hilfspersonal eingeführt werden kann und dessen Einführen für den Patienten weniger belastend ist. Dies gilt aus den genannten Gründen für Koloskope ganz besonders.

Gegenstand der Erfindung ist zum einen eine Endoskopvorrichtung aufweisend folgende Merkmale:
a) ein Endoskoprohr mit einem vorderen Kopfstück,
b) einen flexiblen Stülpschlauch zum Hineinbewegen des Kopfstücks in einen kanalartigen Hohlraum mittels Fluiddrucks,
c) wobei der Stülpschlauch beim Einsatz der Endoskopvorrichtung einen äußeren Bereich, einen mit dem Kopfstück in Eingriff befindlichen Umstülpbereich und einen nach hinten zurückführenden, inneren Bereich aufweist,
d) eine Einrichtung zum Ausüben von Fluidruck in dem gegenüber der Umgebung abgeschlossenen Raum zwischen dem äußeren Bereich und dem inneren Bereich des Stülpschlauchs,
e) und eine Einrichtung zum Ausüben von Fluiddruck in dem Raum zwischen dem Endoskoprohr und dem nach hinten zurückführenden, inneren Bereich des Stülpschlauchs.

Gegenstand der Erfindung ist ferner eine Vorrichtung zum Einführen eines Endoskops in einen kanalartigen Hohlraum, aufweisend folgende Merkmale:
a) einen Stülpschlauch, der beim Einsatz der Vorrichtung einen äußeren Bereich, einen Umstülpbereich zum Eingriff mit einem Kopfstück des Endoskops und einen nach hinten zurückführenden, inneren Bereich aufweist,
b) eine Einrichtung zum Ausüben von Fluiddruck in dem gegenüber der Umgebung abgeschlossenen Raum zwischen dem äußeren Bereich und dem inneren Bereich des Stülpschlauchs,
c) und eine Einrichtung zum Ausüben von Fluidruck in dem Raum an der Innenseite des nach hinten zurückführenden inneren Bereichs des Stülpschlauchs.
   Diese Vorrichtung unterscheidet sich von der im vorstehenden Absatz angegebenen Endoskopvorrichtung dadurch, daß sie das eigentliche Endoskop(rohr) nicht enthält, sondern zur Verwendung zusammen mit dem eigentlichen Endoskop bestimmt ist.

Aus dem Dokument DE-C-28 23 025 ist eine Vorrichtung zum Transport eines Koloskops in den Dickdarm bekannt, die mit einem Stülpschlauch arbeitet. Bei dieser bekannten Vorrichtung kommt jedoch das Vorderende des Koloskops erst dann in eine Position, in der der Dickdarm inspiziert werden kann, wenn der Stülpschlauch vollständig ausgefahren ist. Im Gegensatz hierzu kann man bei der erfindungsgemäßen Vorrichtung den interessierenden Hohlraum, insbesondere den Dickdarm, kontinuierlich der Länge nach während des Hineinbewegens des Endoskops inspizieren.

Aus dem Dokument DE-A-24 06 823 ist ein Katheter bekannt, der mittels eines Stülpschlauchs in einen Körperhohlraum einführbar ist. Es ist erwähnt, daß der Katheter auch eine Fiberglasoptik zur inneren Betrachtung aufweisen kann. Bei einer beschriebenen Ausführungsform weist der Katheter ein Kopfstück auf, mit dem ein Umstülpbereich des Stülpschlauchs in Eingriff ist. Der Stülpschlauch ist jedoch zweifach umgestülpt, so daß drei aufeinanderliegende Stülpschlauchbereiche gebildet sind und kein nach hinten zurückführender, innerer Stülpschlauchbereich vorhanden ist. Hierdurch entstehen beim Hineinbewegen des Katheters übermäßig große Reibungskräfte.

Bei keiner der beiden bekannten Vorrichtungen ist vorgesehen, daß sich in dem Raum zwischen dem Endoskoprohr bzw. dem Katheterrohr und einem nach hinten zurückführenden, inneren Bereich des Stülpschlauchs Druckfluid befindet.

Die erfindungsgemäße Vorrichtung besitzt die weiter vorn genannten, gewünschten Eigenschaften. Das Endoskop wird - abgesehen von der Anfangsphase, in der der Endoskopkopf durch den After geschoben wird - nicht mehr von außen her in den Körper hineingedrückt bzw. hineingeschoben, sondern bewegt sich mittels seines Eigenantriebs in den Körper hinein.

Die Erfindung eignet sich für alle Arten von Endoskopen, ganz besonders aber für Koloskope, also Endoskope zur Exploration des Darms. Die folgende Beschreibung stellt daher auf Koloskope ab, wobei alles Gesagte aber auch analog für andere Endoskope gilt.

Ferner wird betont, daß der erfindungsgemäße Eigenantrieb entweder bei dem Endoskop integriert sein kann oder als Zusatzteil oder Ergänzungsteil für Endoskope bisheriger Konstruktion ausgebildet sein und vertrieben werden kann.

Nach einer besonders bevorzugten Ausgestaltung der Erfindung weist der Eigenantrieb eine mit Fluiddruck beaufschlagbare, vorzugsweise im Querschnitt ringförmige, Kammer zwischen einem äußeren, zurückgestülpten Bereich eines flexiblen Hohlelements und einem inneren Bereich des Hohlelements auf, wobei die Kammer an ihrem dem Umstülpende entgegengesetzten Ende abgedichtet ist, so daß Fluiddruck in der Kammer vorwärtstreibenden, inneren Druck auf das Umstülpende ausübt und dadurch eine zunehmende Länge des Hohlelements unter Wanderung des Umstülpendes in den Darm hineinbewegt wird. Vorzugsweise ist dabei eine gleitende Abdichtung des hinteren Kammerendes vorgesehen. Das Hohlelement hat vorzugsweise eine nur geringe Nachgiebigkeit in Radialrichtung, damit die langgestreckt-ringspaltförmige Kammerkonfiguration erhalten bleibt. Der äußere, zurückgestülpte Bereich des Hohlelements kann an der Darmwand anliegen oder auch nicht.

Der Ringraum zwischen dem inneren Bereich des Hohlelements und dem Endoskoprohr ist während des Hineinbewegens mit Fluiddruck beaufschlagt. Auf diese Weise läßt sich ein Reibungseingriff mit unerwünscht hoher Anpreßkraft zwischen dem inneren Bereich des Hohlelements und dem Außenumfang des Endoskoprohres vermeiden.

Der Dickdarm (Kolon) besteht - vom Anus nach innen fortschreitend - aus dem Rektum, dem Sigma, dem Kolon descendens, dem Kolon transversum, und dem Kolon ascendens, wobei die genannten Dickdarmabschnitte jeweils mit einer Abbiegung von etwa 90° ineinander übergehen. Das Rektum ist digital und durch Rektoskopie vergleichsweise leicht zu untersuchen. Auch die Rektosigmoidoskopie ist noch vergleichsweise unkompliziert durchführbar, da nur eine Darmabbiegung beim konventionellen Einschieben eines Koloskops bewältigt werden muß. Durch Rektosigmoidoskopie lassen sich zwar etwa 60% aller Dickdarmtumoren finden, optimale Ergebnisse erbringt jedoch nur die totale Koloskopie, und diese wird durch die erfindungsgemäße Vorrichtung für eine echte Breitenanwendung, beispielsweise im Sinne einer allgemeinen Vorsorgeuntersuchung ab einem bestimmten Lebensjahr, zugänglich. Aufgrund des erfindungsgemäßen Eigenantriebs folgt das Endoskoprohr bzw. das Führungselement den Darmbiegungen sehr viel leichter als ein konventionelles Koloskop. Das Hineinschieben des Koloskops unter äußerer Krafteinwirkung und das daraus resultierende Andrücken des Endoskoprohres an die Darmwand im Bereich der Darmabbiegungen unter großer Kraft entfallen, abgesehen von der leicht bewältigbaren Passage des Afters ganz zu Beginn des Hineinschiebens. Es ergibt sich eine sehr beträchtliche Personalersparnis bei den Untersuchungen.

Koloskope haben üblicherweise Endoskoprohrlängen etwa im Bereich von 600 bis 1700 mm. Es wird betont, daß sich die meisten Ausführungsformen der erfindungsgemäßen Vorrichtung auch zur nachträglichen Ausrüstung bzw. als Sonderausstattung für die bisher bekannten Koloskope eignen.

Die Erfindung und Ausgestaltungen der Erfindung werden nachfolgend anhand von schematisiert zeichnerisch dargestellten Ausführungsbeispielen noch näher erläutert. Es zeigt:
- Figur 1: eine Endoskopvorrichtung in schematisierter Darstellung im Längsschnitt zur Veranschuaulichung des Funktionsprinzips der Erfindung;
- Figur 2: den hinteren Bereich einer abgewandelten Endoskopvorrichtung zur Veranschaulichung weiterer Einzelheiten.

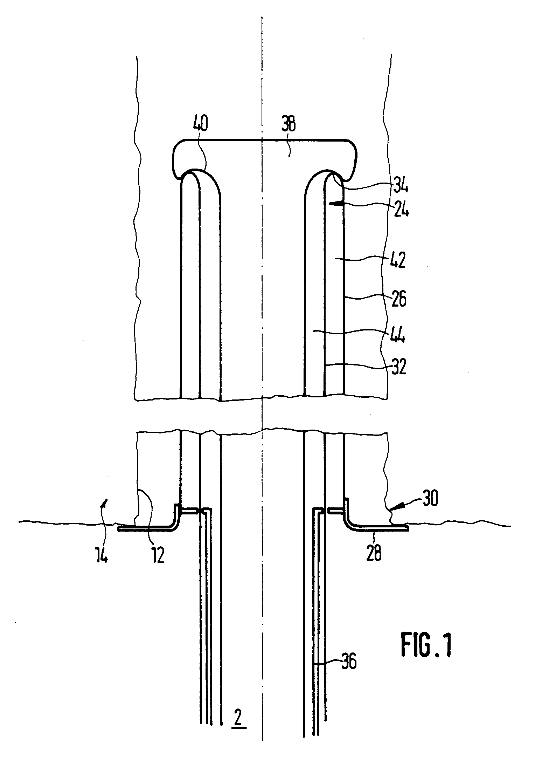

Hauptbestandteil des in Fig. 1 gezeichneten Eigenantriebs ist ein Hohlelement bzw. Stülpschlauch 24 beispielsweise
aus Gummi oder einem verformbaren Kunststoff. Ein Teil der Länge des Stülpschlauchs 24 ist nach außen zurückgestülpt und bildet einen äußeren, zurückgestülpten Bereich 26 des Stülpschlauchs 24. An seinem hinteren Ende ist der zurückgestülpte Bereich 26 mit einem steifen Widerlagerring 28 verbunden, der sich von außen am Anus 30 abstützt. Der nicht zurückgestülpte, innere Bereich 32 des Stülpschlauchs 24 ist aus dem Anus 30 herausgeführt. Die Bereiche 26 und 32 gehen durch einen Umstülpbereich 34, der eine 180°-Umbiegung macht, ineinander über. Innerhalb des inneren Bereichs 32 sitzt, etwa vom Anus 30 aus nach außen führend, eine steife Hülse 36. Das vordere Ende der Hülse 36 ist außen zu einer Dichtlippe geformt. Der Widerlagerring 28 weist an seinem Innenumfang ebenfalls eine Dichtlippe auf. Auf diese Weise ist die Kammer zwischen dem äußeren Bereich 26 des Stülpschlauchs 24 und dem inneren Bereich 32 des Stülpschlauchs 24 (soweit es sich innerhalb des Darms befindet) nach außen hin abgedichtet.

Auf dem Umstülpbereich 34 sitzt ein Kopfstück 38 des Endoskoprohres 2. Das Kopfstück 38 weist rückseitig eine umlaufende, rückwärts-axial weisende Rinne 40 auf. Das Umstülpende 34 sitzt in dieser Rinne 40.

Wenn die beschriebene Kammer 42 unter Fluiddruck gesetzt wird, wird von innen her auf den Umstülpbereich 34 ein Druck ausgeübt, der das Kopfstück 38 vorwärts immer weiter in den Darm hineinbewegt. Während dieser Hineinbewegung wälzt sich gleichsam der Umstülpbereich 34 gleitend in der Rinne 40 ab. Der äußere Bereich 26 bleibt stationär und wird immer länger. Der innere Bereich 32 bewegt sich mit etwa doppelter Geschwindigkeit wie das Endoskoprohr 2 in den Darm hinein und wird ebenfalls immer länger.

Die Bereiche 26 und 32 verlaufen jeweils etwa konzentrisch zum Endoskoprohr 2 und erstrecken sich längs des Darms. Die Bereiche 26 und 32 sollen in Radialrichtung so steif sein, daß sie sich unter dem Fluiddruck innerhalb der Kammer 42 nicht übermäßig nach außen bzw. innen ausbeulen. Zu diesem Zweck kann beispielsweise eine geeignete Faser- oder Gewebeeinlage im Material des Stülpschlauchs 24 vorgesehen sein. Es versteht sich, daß der Widerlagerring 28 gegen den Anus 30 gehalten wird, damit er nicht unter dem Fluiddruck der Kammer 42 nach außen gedrückt wird.

In dem langgestreckten Ringspaltraum 44 zwischen dem inneren Bereich 32 des Stülpschlauchs 24 und dem Außenumfang des Endoskoprohres 2 wird ebenfalls ein Fluiddruck aufgebracht. Dieser hilft dabei, den inneren Bereich 32 auf Abstand von dem Endoskoprohr 2 zu halten, damit hier keine wesentlichen Reibungskräfte entstehen. Dieser Ringraum 44 ist vorn durch den Eingriff zwischen dem Umstülpbereich 34 und der Rinne 40 mindestens im wesentlichen abgedichtet. Kleinere Fluidverluste dort sind nicht störend.

Im Kopfstück 38 ist insbesondere ein nicht eingezeichneter Kamerachip eingebaut, so daß während des Hineinbewegens des Kopfstücks 38 und des Endoskoprohrs 2 die Darminnenwand optisch inspiziert werden kann.

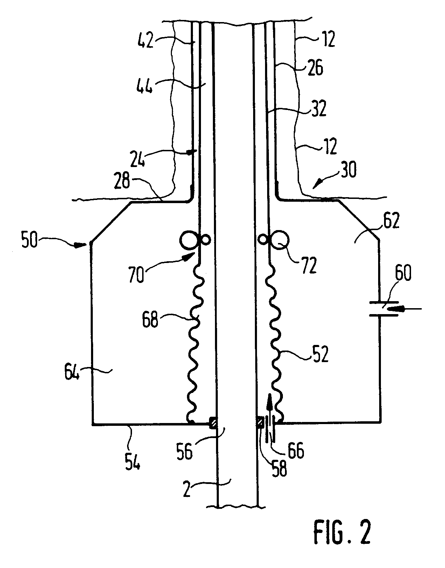

In Fig. 2 ist eine Möglichkeit der abgewandelten Gestaltung des hinteren Endbereichs der Vorrichtung dargestellt.

Der Widerlagerring 28 ist am vorderen Ende einer Druckkammer 50 befestigt. Der am Anus 30 herausgeführte, innere Bereich 32 des Stülpschlauchs 24 geht in der Druckkammer 50 in einen Vorratsbereich 52 des Stülpschlauchs 24 über. In dem Vorratsbereich 52 ist der Stülpschlauch 24 zick-zack-förmig oder gewellt angeordnet. Das hintere, in Fig. 2 untere Ende des Vorratsbereichs 52 ist an der hinteren Wand der Druckkammer 50 befestigt.

Die hintere Wand 54 der Druckkammer 50 weist eine zentrale Öffnung 56 auf, durch die, mit einer Ringdichtung 58 abgedichtet, das Endoskoprohr 2 nach hinten-außen geführt ist. Durch eine Öffnung 60 läßt sich dem Bereich 62 der Druckkammer zwischen dem Vorratsbereich 52 des Stülpschlauchs 24 und der Umfangswand 64 der Druckkammer 50 Fluid unter Druck zuführen. Durch eine Öffnung 66 läßt sich dem Bereich 68 der Druckkammer 50 zwischen dem Endoskoprohr 2 und dem Vorratsbereich 52 des Stülpschlauchs 24 Fluid unter Druck zuführen. Die Zufuhr der Fluide erfolgt beispielsweise mittels geeigneter Pumpen oder von Druckvorratsbehältern her.

Es ist generell bevorzugt, daß dem Raum zwischen dem äußeren Bereich 26 und dem inneren Bereich 32 des Stülpschlauchs 24 ein gasförmiges Druckfluid zum Vorwärtsbewegen des Endoskops zugeführt wird und daß dem Raum 44 zwischen dem inneren Bereich 32 des Stülpschlauchs 24 und dem Endoskoprohr 2 eine Flüssigkeit zum Abstandhalten zugeführt wird. Es ist am besten, wenn die Fluide in den beiden genannten Räumen etwa gleichen Druck haben. Man erkennt in Fig. 2, daß der vorstehend erstgenannte Raum mit dem Bereich 62 der Druckkammer 50 in Verbindung steht und daß der vorstehend zweitgenannte Raum mit dem Bereich 68 der Druckkammer 50 in Verbindung steht.

Es ist alternativ möglich, den Raum 68 in der Druckkammer 50 nicht durch die Öffnung 66 von einer externen Druckquelle unter Druck zu setzen, sondern - mindestens im wesentlichen - über den Vorratsbereich 52 von dem Bereich 62 der Druckkammer 50 her. Dabei muß lediglich darauf geachtet werden, daß der Bereich 68 ein derartiges Anfangsvolumen hat, daß er durch allmähliche Verkleinerung, die mit einer Verlagerung des Vorratsbereichs 52 mehr zum Zentrum hin einhergeht, die allmähliche Vergrößerung des Raums zwischen dem inneren Bereich 32 des Stülpschlauchs 24 und dem Endoskoprohr 2 beim Hineinbewegen des Endoskops in den Darm ausgleicht.

In Fig. 2 ist schließlich eine Antriebseinrichtung zum Zurückziehen des inneren Bereichs 32 des Stülpschlauchs 24 schematisiert eingezeichnet. Diese Einrichtung 70 besteht im wesentlichen aus einer Anzahl von Rollenpaaren 72, die ringförmig verteilt im vorderen Bereich der Druckkammer 50 angebracht sind. Ein Stück außerhalb des Anus 30 ist der innere Bereich 32 des Stülpschlauchs 24 durch den Ring aus Rollenpaaren 72 hindurchgeführt, ehe er in den Vorratsbereich 52 übergeht. Mindestens einige der Rollen der Rollenpaare 72 sind durch nicht eingezeichnete Miniatur-Elektromotoren antreibbar. Beim angetriebenen Drehen dieser Rollenpaare 72 im richtigen Drehsinn wird der innere Bereich 32 des Stülpschlauchs 24 aus dem Darm herausgezogen. Zugleich zieht man am besten an dem Endoskoprohr 2, so daß das Kopfstück 38 im wesentlichen in Kontakt mit dem Umstülpbereich 34 des Stülpschlauchs 24 bleibt. Gleichzeitig kann der Druck im Fluidraum zwischen dem äußeren Bereich 26 und dem inneren Bereich 32 des Stülpschlauchs 24 reduziert werden. Während dieses kontrollierten Zurückfahrens des Endoskops kann die Darmwand nochmals inspiziert werden.

Der Umstülpbereich 34 des Stülpschlauchs 24 muß nicht unbedingt mit einer hinteren Rinne 40 des Kopfstücks 38 in Eingriff sein. Es ist auch möglich, einen Eingriff des Umstülpbereichs 34 mit einer am Außenumfang des Kopfstücks 38 verlaufenden, umlaufenden Rinne vorzusehen. In diesem Fall muß ein äußerer Ring vorgesehen sein, der den Umstülpbereich 34 in der Außenumfangsrinne gegen Verlagerung in Längsrichtung des Endoskoprohrs 2 hält. Dieser Ring kann eine Reihe von ringförmig angeordneten Kugeln oder Rollen aufweisen. Bei dieser Ausführungsform wird das Endoskoprohr 2 mit zurückgezogen, wenn der innere Bereich 32 des Stülpschlauchs 24 zurückgezogen wird.

In den Zeichnungen bezeichnet 14 den Dickdarm und 12 die Darmwand.

Nach einer alternativen Ausbildungsmöglichkeit ist der Vorratsbereich 52 des Stülpschlauchs 24 einschließlich des darin befindlichen Teils des Endoskoprohrs 2 in Windungen oder S-artigen Schleifen mit relativ großem Krümmungsradius in der Druckkammer 50 abgelegt. Der Vorratsbereich 52 muß dann nicht in sich gewellt sein.

Es wird darauf hingewiesen, daß die Antriebseinrichtung gemäß Anspruch 4 auch bei einer Endoskopvorrichtung verwirklicht sein kann, die keine Fluiddruckausübung in dem Raum an der Innenseite des nach hinten zurückführenden, inneren Bereichs des Stülpschlauchs aufweist.

## Patentansprüche

1. Endoskopvorrichtung aufweisend folgende Merkmale:
a) ein Endoskoprohr (2) mit einem vorderen Kopfstück (38),
b) einen flexiblen Stülpschlauch (24) zum Hineinbewegen des Kopfstücks (38) in einen kanalartigen Hohlraum (14) mittels Fluiddrucks,
c) wobei der Stülpschlauch (24) beim Einsatz der Endoskopvorrichtung einen äußeren Bereich (26), einen mit dem Kopfstück (38) in Eingriff befindlichen Umstülpbereich (34) und einen nach hinten zurückführenden, inneren Bereich (32) aufweist,
d) eine Einrichtung zum Ausüben von Fluiddruck in dem gegenüber der Umgebung abgeschlossenen Raum (42) zwischen dem äußeren Bereich (26) und dem inneren Bereich (32) des Stülpschlauchs (24),
e) und eine Einrichtung zum Ausüben von Fluiddruck in dem Raum zwischen dem Endoskoprohr (2) und dem nach hinten zurückführenden, inneren Bereich (32) des Stülpschlauchs (24).

2. Vorrichtung zum Einführen eines Endoskops in einen kanalartigen Hohlraum, aufweisend folgende Merkmale:
a) einen Stülpschlauch (24), der beim Einsatz der Vorrichtung einen äußeren Bereich (26), einen Umstülpbereich (34) zum Eingriff mit einem Kopfstück (38) des Endoskops und einen nach hinten zurückführenden, inneren Bereich (32) aufweist,
b) eine Einrichtung zum Ausüben von Fluiddruck in dem gegenüber der Umgebung abgeschlossenen Raum (42) zwischen dem äußeren Bereich (26) und dem inneren Bereich (32) des Stülpschlauchs (24),
c) und eine Einrichtung zum Ausüben von Fluiddruck in dem Raum an der Innenseite des nach hinten zurückführenden, inneren Bereichs (32) des Stülpschlauchs (24).

3. Vorrichtung nach Anspruch 1, dadurch gekennzeichnet, daß der Umstülpbereich (34) des Stülpschlauchs (24) mit einer rückseitigen, umlaufenden Rinne (40) des Endoskoprohr-Kopfstücks (38) in Eingriff ist.

4. Vorrichtung nach einem der Ansprüche 1 bis 3, gekennzeichnet durch eine Antriebseinrichtung (70) zum Zurückziehen des inneren Bereichs (32) des Stülpschlauchs (24).

5. Vorrichtung nach einem der Ansprüche 1 bis 4, dadurch gekennzeichnet, daß ein Vorratsbereich (52) des Stülpschlauchs (24) in einer Druckkammer (50) angeordnet ist, deren Druck sich zwischen dem äußeren Bereich (26) und dem inneren Bereich (32) des Stülpschlauchs (24) auswirkt.

6. Vorrichtung nach einem der Ansprüche 1 bis 5, dadurch gekennzeichnet, daß das Fluid in dem Raum (42) zwischen dem äußeren Bereich (26) und dem inneren Bereich (32) des Stülpschlauchs (24) ein Gas ist und daß das Fluid in dem Raum an der Innenseite des inneren Bereichs (32) des Stülpschlauchs (24) eine Flüssigkeit ist.

## Claims

1. An endoscope device comprising the following features:
a) an endoscope tube (2) having a forward head piece (38),
b) a flexible eversion tube (24) for moving the head piece (38) into a channel-like cavity (14) by means of fluid pressure,
c) the eversion tube (24) having, when the endoscope device is in use, an outer portion (26), a turning portion (34) engaged with the head piece (38), and a rearwardly extending inner portion (32),
d) a means for exerting fluid pressure in the space (42) between the outer portion (26) and the inner portion (32) of the eversion tube (24), which is sealed from the surroundings,
e) and a means for exerting fluid pressure in the space between the endoscope tube (2) and the rearwardly extending inner portion (32) of the eversion tube (24).

2. A device for introducing an endoscope into a channel-like cavity, comprising the following features:
a) a flexible eversion tube (24) having, when the device is in use, an outer portion (26), a turning portion (34) for engagement with a head piece (38) of the endoscope, and a rearwardly extending inner portion (32),
b) a means for exerting fluid pressure in the space (42) between the outer portion (26) and the inner portion (32) of the eversion tube (24), which is sealed from the surroundings,
c) and a means for exerting fluid pressure in the space on the inside of the rearwardly extending inner portion (32) of the eversion tube (24).

3. A device according to claim 1,
characterized in the the turning portion (34) of the eversion tube (24) is in engagement with a rearward continuous recess (40) of the endoscope tube head piece (38).

4. A device according to any one of claims 1 to 3,
characterized by a driving means (70) for retracting the inner portion (32) of the eversion tube (24).

5. A device according to any one of claims 1 to 4,
characterized in that a supply portion (52) of the eversion tube (24) is disposed in a pressure chamber (50) whose pressure acts between the outer portion (26) and the inner portion (32) of the eversion tube (24).

6. A device according to any one of claims 1 to 5,
characterized in that the fluid in the space (42) between the outer portion (26) and the inner portion (32) of the eversion tube (24) is a gas and that the fluid in the space on the inside of the inner portion (32) of the eversion tube (24) is a liquid.

## Revendications

1. Dispositif endoscopique présentant les caractéristiques suivantes :
a) un tube endoscopique (2) muni d'une pièce de tête antérieure (38),
b) un tuyau retourné flexible (24) pour faire pénétrer la pièce de tête (38) dans une cavité analogue à un canal (14) par pression de fluide,
c) dans lequel, lors de l'utilisation du dispositif endoscopique, le tuyau retourné (24) comporte un domaine externe (26), un domaine de retournement (34) qui est en contact avec la pièce de tête (38) et un domaine interne (32) qui reconduit vers l'arrière,
d) un dispositif pour exercer une pression de fluide dans la cavité (42) fermée par rapport à l'extérieur, entre le domaine externe (26) et le domaine interne (32) du tuyau retourné (24),
e) et un dispositif pour exercer une pression de fluide dans la cavité entre le tube endoscopique (2) et le domaine interne (32) du tuyau retourné (24) qui reconduit vers l'arrière.

2. Dispositif pour insérer un endoscope dans une cavité en forme de canal, présentant les caractéristiques suivantes :
a) un tuyau retourné (24) qui, lors de l'utilisation du dispositif, comporte un domaine externe (26), un domaine de retournement (34) destiné à venir en contact avec une pièce de tête (38) de l'endoscope et un domaine interne (32) qui reconduit vers l'arrière,
b) un dispositif pour exercer une pression de fluide dans la cavité (42) fermée par rapport à l'extérieur entre le domaine externe (26) et le domaine interne (32) du tuyau retourné (24),
c) et un dispositif pour exercer une pression de fluide dans la cavité au niveau du côté interne du domaine interne (32) du tuyau retourné (24) qui reconduit vers l'arrière.

3. Dispositif selon la revendication 1, caractérisé en ce que le domaine de retournement (34) du tuyau retourné (24) est en contact avec une rigole périphérique dorsale (40) de la pièce de tête (38) du tube endoscopique.

4. Dispositif selon l'une des revendications 1 à 3, caractérisé par un dispositif d'actionnement (70) pour tirer vers l'arrière le domaine interne (32) du tuyau retourné (24).

5. Dispositif selon l'une des revendications 1 à 4, caractérisé en ce qu'un domaine de réserve (52) du tuyau retourné (24) est agencé dans une chambre à pression (50) dont la pression s'exerce entre le domaine externe (26) et le domaine interne (32) du tuyau retourné (24).

6. Dispositif selon l'une des revendications 1 à 5, caractérisé en ce que le fluide situé dans la cavité (42) entre le domaine externe (26) et le domaine interne (32) du tuyau retourné (24) est un gaz et en ce que le fluide situé dans la cavité au niveau du côté interne du domaine interne (32) du tuyau retourné (24) est un liquide.
